# EUROPÄISCHE PATENTSCHRIFT

(11) **EP 2 575 612 B1**
(45) Veröffentlichungstag und Bekanntmachung des Hinweises auf die Patenterteilung: **19.08.2020**
(21) Anmeldenummer: 11730881.7
(22) Anmeldetag: 14.03.2011
(51) Int. Cl.: A61B 5/053, A61B 5/00, G01G 19/50

(54) **VORRICHTUNG ZUR MESSUNG VON BIOIMPEDANZEN**
DEVICE FOR MEASURING BIOIMPEDANCES
DISPOSITIF DE MESURE DE BIO-IMPÉDANCE

(30) Priorität: 01.06.2010 DE 102010023122
(43) Veröffentlichungstag der Anmeldung: 10.04.2013
(73) Patentinhaber: seca ag, 4153 Reinach BL 1 (CH)
(72) Erfinder: VOGEL, Sönke, 20149 Hamburg (DE)
(74) Vertreter: Klickow & Wetzel PartGmbB
(86) Internationale Anmeldenummer: PCT/DE2011/000262
(87) Internationale Veröffentlichungsnummer: WO 2011/150903

(56) Entgegenhaltungen:
- EP-A1- 0 998 875
- DE-A1-102006 038 399
- JP-A- 2003 265 429
- JP-A- 2006 175 247
- US-A- 6 165 129
- US-A1- 2004 059 242
- US-A1- 2008 287 816
- US-B1- 6 256 532

## Beschreibung

Die Erfindung betrifft eine Vorrichtung zur Messung von Bioimpedanzen, die jeweils mindestens eine Messelektrode für jeden Fuß und jede Hand einer zu vermessenden Person aufweist und bei der die Elektroden an eine Auswertungseinrichtung angeschlossen sind, sowie die mit mindestens einer Waage zur Bestimmung eines Körpergewichtes der zu vermessenden Person versehen ist.

Derartige Vorrichtungen dienen typischerweise dazu, einen relativen Fettanteil im menschlichen Gewebe zu bestimmen. Aufgrund des relativ geringen Wasseranteils von Fettgewebe ist der elektrische Widerstand derartigen Fettgewebes deutlich größer als der elektrische Widerstand von Muskeln oder Körperflüssigkeiten. Über eine entsprechende Widerstand- oder Impedanzmessung in Kombination mit einer Gewichtsmessung kahn deshalb dieser Fettanfeil bestimmt werden.

Wesentlich für die Erreichung genauer Messungen ist es, dass zumindest der wesentliche Teil des Körpers vom Messstrom durchflossen wird. Für genaue Messungen sind deshalb sowohl im Bereich der Füße als auch im Bereich der Hände Elektroden erforderlich.

Für eine schnelle und vom Benutzer als angenehm empfundene Messung ist es erforderlich, den Kontakt mit den Elektroden einfach und angenehm herzustellen. Es wird deshalb angestrebt, dass die zu vermessende Person sich mit den Füßen auf die jeweils zugeordnete Elektrode stellt und die für die Hände vorgesehenen Elektroden ergreift. Relativ unproblematisch ist hierbei ein korrekter Kontakt zwischen den Füßen und den Fußelektroden, wenn eine korrekte Ausrichtung der zu vermessenden Person unterstützt wird. Ein korrekter Kontakt mit den zu ergreifenden Handelektroden setzt eine korrekte relative Positionierung zwischen den Händen und den Elektroden voraus.

Aus der US 6256532 B1 ist bereits eine Vorrichtung zur Messung von Bioimpedanzen bekannt, die mit Messelektroden für die Füße und die Hände einer zu vermessenden Person aufweist. Die Elektroden sind an einer Auswertungseinrichtung angeschlossen.

Ähnliche Vorrichtungen werden auch in der JP 2003265429 A sowie der EP 0998857 A1 beschrieben.

Aus der US 2004/0059242 A1 ist bereits eine Vorrichtung zur Messung von Bioimpedanzen bekannt, bei der im Bereich jeden Fußes und jeder Hand eines Patienten Messelektroden angeordnet werden. Zusätzlich wird das Körpergewicht einer Person über eine Waage bestimmt und die Handelektroden sind im Bereich eines Tragelementes angeordnet. Gemäß einem Ausführungsbeispiel weist das Tragelement zwei Handgriffe auf, an denen die Elektroden positioniert sind. Gemäß einem weiteren Ausführungsbeispiel befinden sich die Handelektroden im Bereich eines U-förmigen Trägers, der an einer Tragsäule montiert ist. Gemäß einem weiteren Ausführungsbeispiel erfolgt die Anordnung im Bereich einer Tragstruktur ähnlich zu einem Stuhl.

Aufgabe der vorliegenden Erfindung ist es, eine Vorrichtung der einleitend genannten Art derart zu konstruieren, dass eine korrekte Positionierung der Elektroden relativ zur vermessenden Person unterstützt wird.

Diese Aufgabe wird erfindungsgemäß durch die Merkmalskombination von Patentanspruch Nr. 1 gelöst.

Durch die Anordnung der Elektroden im Bereich eines positionierbaren Tragelementes können eine Mehrzahl von Vorteilen erreicht werden. Zum einen ist es möglich, die Positionierung der Elektroden an die Körpergröße, die konkrete Anatomie des Benutzers sowie die Körperhaltung des Benutzers anzupassen. Diese Anpassbarkeit führt zum einen zu einem guten elektrischen Kontakt zwischen den Elektroden und den Händen des Benutzers, darüber hinaus wird auch eine angenehme Benutzungshaltung unterstützt. Ebenfalls ist es möglich, unter Verwendung des Tragelementes eine korrekte Positionierung des Benutzers relativ zur Waage sowie zu den Fußelektroden zu unterstützen. Auch hierdurch wird eine korrekte Messung erreicht.

Eine Anpassung an eine Körpergröße des Benutzers kann dadurch erfolgen, dass das Tragelement höhenpositionierbar angeordnet ist.

Eine gespreizte Armhaltung wird dadurch unterstützt, dass das Tragelement mit einer horizontalen Richtungskomponente positionierbar ist.

Eine angenehme Griffposition und/oder eine Verstellung wird dadurch unterstützt, dass das Tragelement verdrehbar angeordnet ist.

Vorgegebene Positionierbahnen können dadurch definiert werden, dass das Tragelement verschieblich entlang eines Führüngselementes angeordnet ist.

Eine robuste mechanische Ausführungsform wird dadurch bereitgestellt, dass das Führungselement an einer Standsäule befestigt ist, die eine Anzeige trägt.

Darüber hinaus trägt es zu einer mechanischen Belastbarkeit bei, dass das Führungselement im Bereich seiner der Standsäule abgewandten Enden von Streben abgestützt ist.

Gemäß einer Ausführungsform ist vorgesehen, dass das Führungselement relativ zur Standsäule höhenpositionierbar angeordnet ist.

Eine besonders hohe Benutzungsfreundlichkeit wird dadurch erreicht, dass der Standsäule abgewandte Enden des Führungselementes in einer vertikalen Richtung niedriger als der der Standsäule zugewandte Bereich des Führungselementes angeordnet sind.

Eine gute Stützfunktion für den Benutzer bei gleichzeitig optimaler Verstellmöglichkeit für die Elektroden wird dadurch erreicht, dass sich das Führungselement halbkreisartig erstreckt.

Besonders genaue Messergebnisse werden dadurch erreicht, dass die Auswertungseinrichtung sowohl zur Auswertung eines gemessenen Wechselstromes als auch zur Auswertung einer gemessenen Wechselspannung ausgebildet ist.

Die vorstehend definierte Positionierbarkeit des mindestens einen Tragelementes für die Elektroden kann unterschiedlich realisiert werden. Gemäß einer ersten Ausführungsvariante ist für die jeder Hand eines Benutzers zugeordneten Elektroden jeweils ein Tragelement vorgesehen, das entweder entlang des Führungselementes oder gemeinsam mit dem Führungselement positionsveränderlich angeordnet ist. Durch eine entsprechende Positionsveränderung der Tragelemente wird die optimale Elektrodenpositionierung eingenommen.

Gemäß einer anderen Ausführungsvariante sind für jede Hand des Benutzers mindestens zwei Tragelemente für die Elektroden vorgesehen, wobei die Tragelemente relativ zum Führungselement unbeweglich angeordnet sind. Beispielsweise können entlang der Längserstreckung des Führungselementes für jede Hand des Benutzers drei Tragelemente, vorzugsweise für jeweils zwei Elektroden, angeordnet sein. Der Benutzer ergreift hierbei jeweils die Elektroden, die in Relation zu seiner Anatomie am günstigsten positioniert sind. Ein derartiges Umgreifen in Abhängigkeit von der konkreten Anatomie des Benutzers wird insbesondere dadurch unterstützt, dass das Führungselement ausgehend von der Standsäule nach unten geneigt verläuft und/oder einen bogenartigen Verlauf besitzt.

Hinsichtlich der Neigung des Führungselementes relativ zur Horizontalen erweist sich für den entsprechenden Winkel ein Bereich von 10 Grad bis 40 Grad als zweckmäßig. Bevorzugt ist ein Bereich von 20 Grad bis 30 Grad. Beim vorliegenden Ausführungsbeispiel ist ein Winkel von etwa 25° realisiert.

In den Zeichnungen sind Ausführungsbeispiele der Erfindung schematisch dargestellt. Es zeigen:
- Fig. 1: eine perspektivische Darstellung einer Vorrichtung mit relingartigem Führungselement für die Handelektroden sowie im Bereich einer Waage angeordneten Fußelektroden,
- Fig. 2: eine gegenüber der Ausführungsform in Fig. 1 abgewandelte Ausführungsform mit zusätzlichen seitlichen Tragarmen für die Elektroden,
- Fig. 3: eine gegenüber Fig. 1 abgewandelte Ausführungsform mit verkürzten Führungselementen für die Elektroden,
- Fig. 4: eine schematische Darstellung zur Veranschaulichung sowohl einer Verdrehbarkeit als auch einer Verschieblichkeit des Tragelementes für die Elektroden relativ zu einem Führungselement,
- Fig. 5: eine weitere Darstellung zur Veranschaulichung einer Positionierbarkeit des Tragelementes für die Elektroden,
- Fig. 6: eine perspektivische Darstellung zur Veranschaulichung einer Höhenpositionierbarkeit des Tragelementes und
- Fig. 7: eine schematische Darstellung zur Veranschaulichung des Messprinzips.

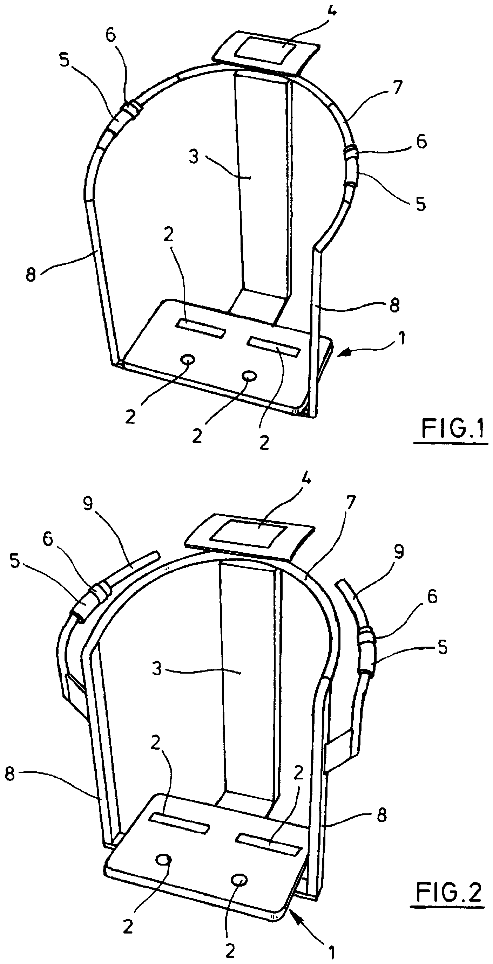

Gemäß der Ausführungsform in Fig. 1 weist die Vorrichtung zur Messung von Bioimpedanzen eine Waage (1) auf, die mit Fußelektroden (2) versehen ist. Beim dargestellten Ausführungsbeispiel sind für jeden Fuß der zu vermessenden Person zwei Fußelektroden (2) vorgesehen.

Im Bereich einer Standsäule (3) ist eine Anzeige (4) angeordnet. Die Anzeige (4) kann als ein Display ausgebildet sein. Darüber hinaus können im Bereich der Standsäule (3) auch Bedienelemente vorgesehen sein. Die Anordnung der Anzeige (4) erfolgt auf einer gut von einem Benutzer ablesbaren Höhe.

Im Bereich von Tragelementen (5) sind Handelektroden (6) angeordnet. Vorzugsweise sind für jede Hand des Benutzers zwei Handelektroden (6) verwendet.

Die Tragelemente (5) sind positionierbar entlang eines Führungselementes (7) angeordnet. Beim dargestellten Ausführungsbeispiel ist das Führungselement (7) an der Standsäule (6) befestigt und weist einen halbkreisartigen Verlauf auf. Fig. 1 zeigt eine Ausführungsform, bei der der Standsäule (3) abgewandt angeordnete Enden des Führungselementes (7) von Streben (8) relativ zu einer Standfläche abgestützt sind. Vorzugsweise sind die im Bereich der Streben (8) angeordneten Enden des Führungselementes (7) in vertikaler Richtung auf einem niedrigeren Niveau positioniert als derjenige Bereich des Führungselementes (7), der mit der Standsäule (3) verbunden ist. Das Führungselement (7) und/oder die Streben (8) können aus einem rohrartigen Profil hergestellt sein. Fig. 1 zeigt eine relingartige Anordnung des Führungselementes (7).

Fig. 2 zeigt eine gegenüber Fig. 1 abgewandelte Ausführungsform. Im Unterschied zu Fig. 1, bei der die Streben (8) in einem seitlichen Bereich der Waage (1) und benachbart zu einer der Standsäule (3) abgewandten Vorderkante der Waage (1) positioniert sind, erfolgt die Anordnung gemäß Fig. 2 in einem seitlichen mehr mittleren Bereich der Waage (1). Darüber hinaus sind die Tragelemente (5) im Unterschied zu Fig. 1 im Bereich seitlicher zusätzlicher Tragarme (9) des Führungselementes (7) angeordnet. Der zur Ausführungsform gemäß Fig. 1 ähnliche Bereich des Führungselementes (7) dient gemäß der Ausführungsform in Fig. 2 nur als Halteelement für den Benutzer und unterstützt eine korrekte körperliche Ausrichtung des Benutzers.

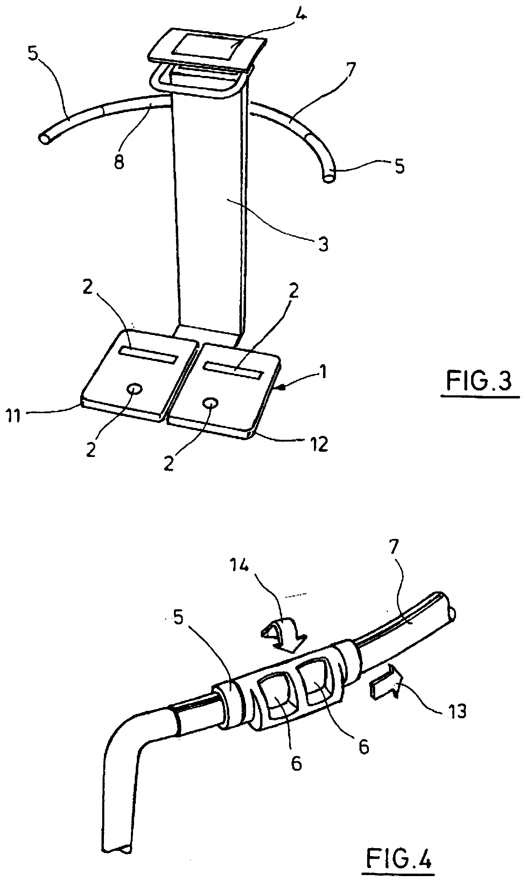

Gemäß der Ausführungsform in Fig. 3 besteht das Führungselement (7) nur aus zwei seitlich über die Standsäule (3) vorstehenden Armen. Zur Erhöhung der Bediensicherheit ist ein zusätzlicher Handgriff (10) verwendet. Die Waage (1) ist in zwei Segmente (11, 12) unterteilt.

Fig. 4 veranschaulicht eine Möglichkeit zur Positionierung des Tragelementes (5) relativ zum Führungselement (7). Das Tragelement (5) ist sowohl in einer Längsrichtung (13) als auch in einer Umfangsrichtung (14) des Führungselementes (7) positionierbar. Hierdurch kann eine an die jeweilige Anatomie des Benutzers optimale Positionierung erreicht werden.

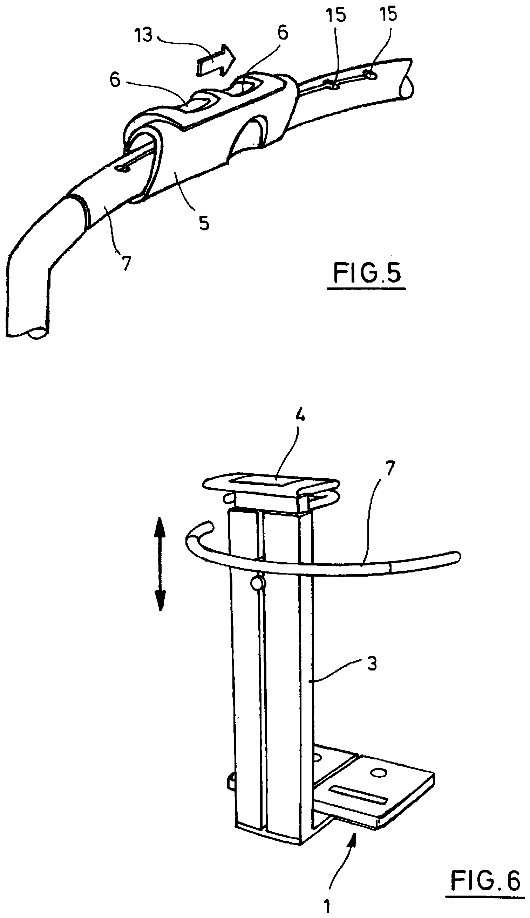

Gemäß der Ausführungsform in Fig. 5 sind im Bereich des Führungselementes (7) Rastungen (15) für das Tragelement (5) angeordnet. Die Rastungen (15) stellen definierte Positionierungen für das Tragelement (5) bereit und erleichtern eine relativ zur Standsäule (3) gleichartige Anordnung der Handelektroden (6).

Fig. 6 veranschaulicht eine Höhenpositionierung eines Führungselementes (7) relativ zur Standsäule (3) bei einer Ausführungsform ähnlich zur Ausführungsform in Fig. 3.

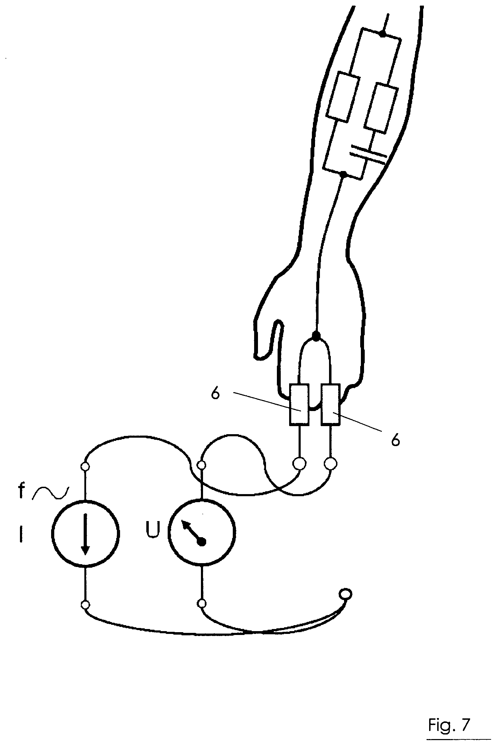

Fig. 7 veranschaulicht das Messprinzip. An die Handelektroden (6) wird eine Wechselspannung angelegt und es wird sowohl der sich ergebende Wechselstrom als auch die an den Handelektroden (6) anliegende Wechselspannung ermittelt. Durch diese Aufspaltung der Messwege für den Wechselstrom und die Wechselspannung wird eine Verfälschung des Messergebnisses durch die Impedanz der Haut im Kontaktbereich der Handelektroden (6) vermieden.

Grundsätzlich wird durch die Anordnung des Tragelementes (5) im Bereich des Führungselementes (7) und durch die Formgebung des Führungselementes (7) erreicht, dass die zu vermessende Person während der gesamten Durchführung der Messung eine leicht seitlich abgespreizte Armhaltung aufweist. Hierdurch wird ein seitlicher Kontakt zwischen den Armen und dem Körper des Benutzers vermieden, der durch die Bereitstellung zusätzlicher elektrischer Leitungswege das Messergebnis verfälschen würde. Für diese seitliche Abspreizung erweist sich die vorstehend beschriebene Ausformung des Führungselementes (7) entsprechend einer Handreling und die Neigung des Verlaufes des Führungselementes (7) relativ zur horizontalen Richtung als vorteilhaft. Unabhängig von der konkreten Größe und der Armlänge eines Benutzers kann der Benutzer seine Hände in eine für die Durchführung der Messung optimale Position überführen.

## Patentansprüche

1. Vorrichtung zur Messung von Bioimpedanzen, die jeweils mindestens eine Messelektrode für jeden Fuß und jede Hand einer zu vermessenden Person aufweist und bei der die Elektroden an eine Auswertungseinrichtung angeschlossen sind, sowie die mit mindestens einer Waage zur Bestimmung eines Körpergewichtes der zu vermessenden Person versehen ist, wobei die für einen Kontakt mit den Händen der zu vermessenden Person vorgesehenen Handelektroden (6) im Bereich von mindestens zwei positionierbaren Tragelementen (5) angeordnet sind und wobei jedes der Trageelemente (5) mit zwei Handelektroden (6) versehen ist, wobei jedes der Tragelemente (5) verdrehbar und verschieblich entlang eines Führungselementes (7) angeordnet ist und wobei sich das Führungselement (7) halbkreisartig erstreckt.

2. Vorrichtung nach Anspruch 1, **dadurch gekennzeichnet, dass** jedes der Tragelemente (5) höhenpositionierbar angeordnet ist.

3. Vorrichtung nach Anspruch 1 oder 2, **dadurch gekennzeichnet, dass** jedes der Tragelemente (5) mit einer horizontalen Richtungskomponente positionierbar ist.

4. Vorrichtung nach einem der Ansprüche 1 bis 3, **dadurch gekennzeichnet, dass** das Führungselement (7) an einer Standsäule (3) befestigt ist, die eine Anzeige (4) trägt.

5. Vorrichtung nach Anspruch 4, **dadurch gekennzeichnet, dass** das Führungselement (7) im Bereich seiner der Standsäule (3) abgewandten Enden von Streben (8) abgestützt ist.

6. Vorrichtung nach einem der Ansprüche 4 oder 5, **dadurch gekennzeichnet, dass** das Führungselement (7) relativ zur Standsäule (3) höhenpositionierbar angeordnet ist.

7. Vorrichtung nach einem der Ansprüche 4 bis 6, **dadurch gekennzeichnet, dass** der Standsäule (3) abgewandte Enden des Führungselementes (7) in einer vertikalen Richtung niedriger als der der Standsäule (3) zugewandte Bereich des Führungselementes (7) angeordnet sind.

8. Vorrichtung nach einem der Ansprüche 1 bis 7, **dadurch gekennzeichnet, dass** die Auswertungseinrichtung sowohl zur Auswertung eines gemessenen Wechselstromes als auch zur Auswertung einer gemessenen Wechselspannung ausgebildet ist.

## Claims

1. Device for measuring bioimpedances, which has at least one measuring electrode for each foot and at least one for each hand of a person who is to be measured, and in which the electrodes are connected to an evaluation unit, and which is provided with at least one balance for determining a body weight of the person who is to be measured, wherein the hand electrodes (6), provided for contact with the hands of the person who is to be measured, are arranged in the region of at least two positionable support elements (5), and wherein each of the support elements (5) is provided with two hand electrodes (6), wherein each of the support elements (5) is arranged rotatably and displaceably along a guide element (7), and wherein the guide element (7) extends in the shape of a semicircle.

2. Device according to Claim 1, **characterized in that** each of the support elements (5) is arranged to be positionable in height.

3. Device according to Claim 1 or 2, **characterized in that** each of the support elements (5) is positionable with a horizontal directional component.

4. Device according to one of Claims 1 to 3, **characterized in that** the guide element (7) is secured to a vertical column (3), which supports a display (4).

5. Device according to Claim 4, **characterized in that** the guide element (7) is supported by struts (8) in the region of its ends directed away from the vertical column (3) .

6. Device according to either of Claims 4 and 5, **characterized in that** the guide element (7) is arranged to be positionable in height relative to the vertical column (3).

7. Device according to one of Claims 4 to 6, **characterized in that** ends of the guide element (7) directed away from the vertical column (3) are lower in a vertical direction than the region of the guide element (7) directed towards the vertical column (3).

8. Device according to one of Claims 1 to 7, **characterized in that** the evaluation unit is designed both to evaluate a measured alternating current and to evaluate a measured alternating voltage.

## Revendications

1. Dispositif de mesure de bioimpédances qui présente respectivement au moins une électrode de mesure pour chaque pied et chaque main d'une personne faisant l'objet de la mesure et dans lequel les électrodes sont connectées à un dispositif d'évaluation, et qui est muni d'au moins une balance pour déterminer un poids corporel de la personne faisant l'objet de la mesure,
dans lequel les électrodes de main (6) prévues pour un contact avec les mains de la personne faisant l'objet de la mesure sont disposées au niveau d'au moins deux éléments de support (5) positionnables, et chacun des éléments de support (5) étant muni de deux électrodes de main (6), chacun des éléments de support (5) étant disposé de manière pivotante et déplaçable le long d'un élément de guidage (7) et l'élément de guidage (7) s'étendant en demi-cercle.

2. Dispositif selon la revendication 1, **caractérisé en ce que** chacun des éléments de support (5) est disposé en étant positionnable en hauteur.

3. Dispositif selon la revendication 1 ou 2, **caractérisé en ce que** chacun des éléments de support (5) est positionnable avec une composante directionnelle horizontale.

4. Dispositif selon l'une quelconque des revendications 1 à 3, **caractérisé en ce que** l'élément de guidage (7) est fixé à une colonne sur pied (3) portant un affichage (4).

5. Dispositif selon la revendication 4, **caractérisé en ce que** l'élément de guidage (7) est soutenu par des entretoises (8) au niveau de ses extrémités détournées de la colonne sur pied (3).

6. Dispositif selon l'une quelconque des revendications 4 et 5, **caractérisé en ce que** l'élément de guidage (7) est disposé de manière positionnable en hauteur par rapport à la colonne sur pied (3).

7. Dispositif selon l'une quelconque des revendications 4 à 6, **caractérisé en ce que** des extrémités, détournées de la colonne sur pied (3), de l'élément de guidage (7) sont disposées plus bas dans la direction verticale que la zone, tournée vers la colonne sur pied (3), de l'élément de guidage (7).

8. Dispositif selon l'une quelconque des revendications 1 à 7, **caractérisé en ce que** le dispositif d'évaluation est réalisé à la fois pour évaluer un courant alternatif mesuré et pour évaluer une tension alternative mesurée.
